(19) Europäisches Patentamt
European Patent Office
Office européen des brevets

(11) Publication number: 0 008 939
B1

(12) EUROPEAN PATENT SPECIFICATION

(45) Date of publication of patent specification: 16.02.83

(21) Application number: 79301816.9

(22) Date of filing: 04.09.79

(51) Int. Cl.³: C 09 D 5/18, D 06 M 15/64, C 09 K 3/28, C 07 D 251/00

# (54) Intumescent composition.

(30) Priority: 05.09.78 US 939628

(43) Date of publication of application: 19.03.80 Bulletin 80/6

(45) Publication of the grant of the patent: 16.02.83 Bulletin 83/7

(84) Designated Contracting States: DE FR GB

(56) References cited:
DE - A - 2 110 415
DE - A - 2 137 356
GB - A - 835 581
US - A - 2 971 929

(73) Proprietor: GREAT LAKES CHEMICAL CORPORATION
Highway 52 Northwest
West Lafayette Indiana 47906 (US)

(72) Inventor: Smith, Ray Eugene
Save Inn Room 149 PO Box 22127
Lake Buena Vista Florida 32830 (US)
Inventor: Shukla, Jayendra G.
1960 Traver Road Apt. No 206
Ann Arbor, Michigan 48105 (US)
Inventor: Daniher, Francis Andrew
3512 Paisley Court
Ann Arbor, Michigan (US)
Inventor: Nicholson, Richard Ralph
1625 Hatcher Crescent
Ann Arbor, Michigan (US)

(74) Representative: McCall, John Douglas et al,
W.P. THOMPSON & CO. Coopers Building Church Street
Liverpool L1 3AB (GB)

EP 0 008 939 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

## Intumescent composition

This invention relates to a novel intumescent composition containing methylated methylol melamine, an hydroxy-containing organo-phosphorus compound, an organo-phosphorous acid, and water.

It is difficult to impart durable flame retardance to a wood substrate. Wood substrates present a flameproofing problem which is materially different from that presented by fibrous hydrophilic organic substrates. According to U.S. patent 2,927,050 in the latter substrate "... substantially independent fibers are tangled together, leaving interstices capable of being filled by an aqueous medium by capillary action between all of their surfaces. The individual fibers contain a relatively small amount of cellulose, and the materials composed of them have a relatively low ignition temperature." In wood substrates, however, "... the cellulosic fibers are bonded together to form a relatively impenetrable block susceptible to little capillary action. A piece of wood has small surface area in relation to the amount of surface area it contains and has a relatively high ignition temperature." Because of these factors, "... a flameproofing agent which flameproofs fibrous hydrophilic organic materials is not likely to flameproof wood because its capacity to inhibit burning is likely to be destroyed by the time it is heated to the ignition temperature of the wood and/or because of the difficulty of causing a non-volatile substance to penetrate into the volume of a block of wood."

Impregnation of a wood substrate with a fire-retardant is known in the art. U.S. patent 3,398,019 teaches that this method must be used to impart a satisfactory degree of flame retardancy to wood fiber insulation, stating that "as far as is known only by the use of a chemical retardant which impregnates the board can a commercially acceptable building material be produced which is capable of securing a nonflammable rating." U.S. patent 4,049,849 teaches that this method, although well known, presents several substantial problems. According to this patent, the use of a wood substrate impregnated with a fire-retardant salt is restricted to low humidity applications "... due to the water solubility and hygroscopicity of most known fire retardant salts." Thus, "... if an ammonium phosphate-impregnated wood substrate is exposed to high (greater than 90 percent) humidity at ambient temperature, in approximately 3 days the fire retardant impregnant (salt) will leach therefrom.... The salt will absorb sufficient water vapor to enable it to migrate to the wood substrate surface. Not only does this leaching deplete the salt content of the wood substrate, rendering it less fire resistant, but it also severely disfigures the wood substrates surface...."

Many of the flame retardants which are used to impregnate wood substrates are acidic and hygroscopic. U.S. patent 3,811,992 teaches that the use of these compositions dehydrates the wood and that "... the wood, in order to make up for the dehydration caused by these hygroscopic agents, must absorb water from the moisture in the ambient air." Such moisture absorption will tend to discolor the plywood and rust and stain connecting members such as nails; and it "... dilutes the amount of agent used in the wood ... and causes the wood to be damp and moist thus not only corroding the wood but subjecting it to attack from insects and the like...."

Those in the art have attempted to impart durable flame retardance to wood substrates by applying intumescent compositions to them. Many intumescent compositions have been tested; U.S. patent 3,668,121 correctly states that only a few of them are of any value. Many of them produce excessive smoke and/or toxic gaseous pyrolysis products. According to U.S. patent 3,769,074, most of these prior art intumescent compositions are "... characterized by the disadvantages of high cost, low spreading rate, relatively poor efficiency, and poor weatherability". U.S. patent 3,513,114 teaches that prior art intumescent coating compositions "... exhibit the distinct disadvantage of either or both failing to maintain a coating film which will withstand repeated scrubbing or washing and thus exhibit wet abrasion resistant properties and/or failing to perform their intended function, that is, to intumesce, and thus fire retard after repeated scrubbing or washing ". U.S. patent 3,535,130 teaches that "... conventional intumescent paints are usually sensitive to attack by water...." U.S. patent 3,654,190 discloses that prior art intumescent compositions are water permeable and tend to degrade when exposed to moist environments.

U.S. patent 3,513,114 discloses that the problems presented by the prior art intumescent compositions cannot be solved merely by replacing the water soluble flame retardant agents they contain with water insoluble additives, for such substitution does not necessarily increase the wet abrasion resistance properties of the compositions. Furthermore, such a substitution will present a new set of problems if the water insoluble additive must be dissolved in an organic solvent; for many dangers are created by the use of the common organic solvents. Toluene, for example, is a fire hazard and an explosion hazard when exposed to heat and flame; and it emits toxic fumes. Methylene chloride is very dangerous to the eyes. Benzene is highly flammable, causes leukemia, and it is a known carcinogen. Acetone is a fire hazard when exposed to either heat or flame. Methanol possesses narcotic properties and exerts a toxic effect upon the nervous system; once it is absorbed into the body, it is eliminated very slowly and, thus, is a cumulative poison. The use of almost any of the common organic solvents will present some hazard.

U.S. patent 3,654,190 teaches that many prior art intumescent coating compositions are soft and "prone to chip with rough handling".

Other prior art considered by the applicants during the preparation of this application includes U.S. patent 2,711,998 (a composition containing trimethylol melamine and the ammonium salt of halopropyl phosphoric acid used to flame retard cotton), U.S. patent 2,676,162 (an intumescent coating for wood containing organic solvent, methylated methylol melamine, the reaction product of ammonia and phosphoryl chloride, and a film-forming condensation product), U.S patent 3,449,161 (fire-retardancy can be incorporated into paint compositions using organo-phosphorous amides), U.S. patent 3,635,970 (melamine phosphate is especially useful in intumescent paint compositions), U.S. patent 4,026,810 (an intumescent flame retardant prepared by reacting, e.g., phosphoric oxide, phosphoric acid, pentaerythritol, and melamine and thereafter curing the reaction product by heating to evolve gaseous products), U.S. patent 2,582,961 (an aqueous flame retardant for cellulosic fiber containing, e.g., methylated methylol melamine, methylol dicyandiamide, and an oxygen-containing acid of phosphorus), U.S. patent 2,661,342 (flameproofing of cellulosic materials with a resinous aminoplast condensation product such as melamine and a water-soluble nitrogen- and phosphorus-containing product), U.S. patent 3,023,176 (a water-soluble hardenable condensation product which is prepared by reacting a methylol compound of the amonotriazine group, an aliphatic compound containing a chain of at least 7 carbon atoms and a reactive hydrogen bound to a hetero atom, and a compound that is capable of introducing atomic groupings that raise the hydrophility in a non-ionic manner), U.S. patent 3,101,278 (methylol-phosphorus polymers which have nitrogen atoms incorporated into them are excellent flame retardants and are suitable for treating cellulosic materials), and U.S. patent 3,332,240 (an aqueous solution for flameproofing cotton fiber containing a salt of hydroxylamine and melamine resin).

It does not appear that the prior art describes an aqueous composition which can impart durable flame retardance to wood substrates even after repeated exposure to water, which will form a coating film which will withstand repeated scrubbing or washing, and which will substantially decrease the amount of noxious fumes generated during pyrolysis of the treated wood substrate.

Applicants have discovered a unique intumescent composition which is substantially superior to the prior art compositions. None of the individual ingredients of applicants' composition intumesce when exposed to an open flame; however, the coatings formed from this composition intumesce during pyrolysis.

In accordance with this invention, there is provided an intumescent composition containing a cyclic nitrogen compound, a hydroxy-containing organo-phosphorus compound, an organo-phosphorus acid, and water, wherein:

1) said cyclic nitrogen compound is

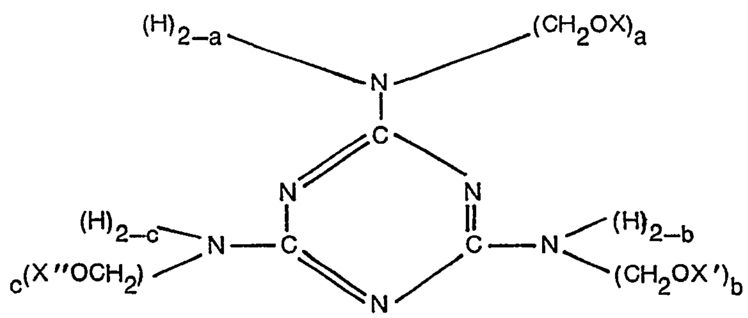

wherein a, b, and c are integers selected from the group consisting of 1 and 2, a plus b plus c equal 3 to 6, and X, X′, and X″ are independently selected from the group consisting of hydrogen and $-CH_3$;

2) said phosphorus compound is selected from the group consisting of

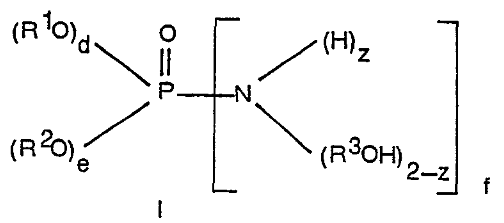

I

and

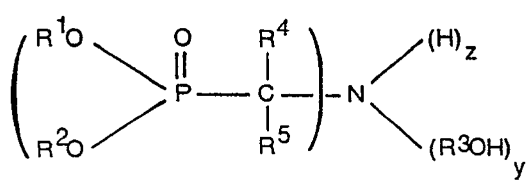

II

and mixtures thereof, wherein d is an integer of from 0 to 2, e is an integer of from 0 to 2, f is an integer of from 1 to 3, and d plus e plus f equal 3; z is an integer of 0 to 1; x is an integer of 1 to 2, y is an integer of 1 to 2, and x plus y plus z equal 3; $R^1$, $R^2$, and $R^3$ are selected from group consisting of alkyl radicals containing from 1 to 6 carbon atoms and haloalkyl radicals containing from 2 to 6 carbon

atoms, provided that when $R^3$ is haloalkyl it contains at least 3 carbon atoms; $R^4$ and $R^5$ are selected from the group consisting of hydrogen, alkyl radicals, and haloalkyl radicals, wherein said radicals contain from 1 to 6 carbon atoms; and the total number of carbon atoms in the $R^1$, $R^2$, $R^3$, $R^4$, and $R^5$ groups does not exceed 14; and

3) said acid is selected from the group consisting of

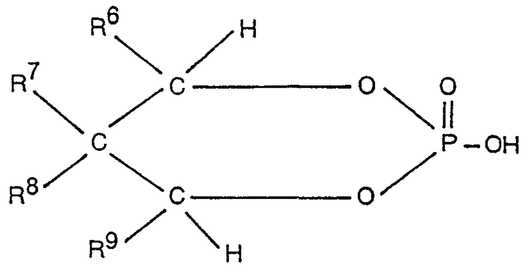
,
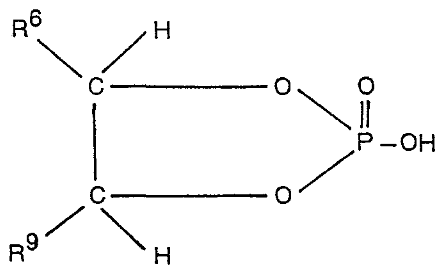
,

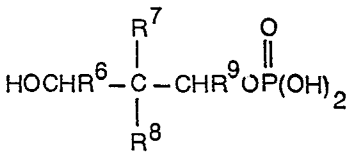
,
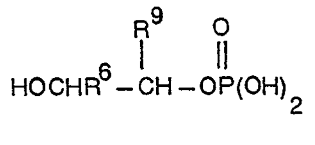
,
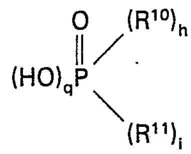

and mixtures thereof, wherein $R^6$, $R^7$, $R^8$ and $R^9$ are independently selected from the group consisting of hydrogen, alkyl of from 1 to 3 carbon atoms, haloalkyl of from 1 to 3 carbon atoms, and hydroxyalkyl of from 1 to 3 carbon atoms, provided that the total number of carbon atoms in the $R^6$, $R^7$, $R^8$, and $R^9$ groups does not exceed 5; wherein q is an integer of from 1 to 2, h and i are integers independently selected from the group consisting of 0, 1, and 2, and q plus h plus i equals 3; and wherein $R^{10}$ and $R^{11}$ are independently selected from the group consisting of alkyl of from 1 to 6 carbon atoms, haloalkyl of from 1 to 6 carbon atoms and from 1 to 3 halogen atoms, hydroxy-polyalkyleneoxy containing 2 to 6 carbon atoms and 2 to 6 oxygen atoms, alkoxy of from 1 to 6 carbon atoms, hydroxyalkoxy of from 2 to 6 carbon atoms, haloalkoxy of from 2 to 6 carbon atoms and from 1 to 3 halogen atoms,

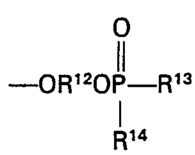

wherein $R^{12}$ is selected from the group consisting of alkylene of from 2 to 6 carbon atoms, haloalkylene of from 3 to 6 carbon atoms and from 1 to 3 halogen atoms, polyalkyleneoxy containing 2 to 6 carbon atoms and 1 to 5 oxygen atoms, and hydroxyalkylene of from 3 to 6 carbon atoms and 1 to 4 hydroxyls, and $R^{13}$ and $R^{14}$ are independently selected from the group consisting of hydrogen, hydroxyl, alkyl of from 1 to 6 carbon atoms, haloalkyl of from 1 to 6 carbon atoms and from 1 to 3 halogen atoms, hydroxy-polyalkyleneoxy containing 2 to 6 carbon atoms and 2 to 6 oxygen atoms, alkoxy of from 1 to 6 carbon atoms, hydroxyalkoxy of from 2 to 6 carbon atoms, and haloalkoxy of from 2 to 6 carbon atoms and 1 to 3 halogen atoms, provided that the total number of carbon atoms in the $R^{10}$ and $R^{11}$ groups does not exceed 8.

The intumescent coating composition of this invention contains a cyclic nitrogen compound. It is preferred that this composition contain 30 to 60 percent (by weight) of said cyclic nitrogen compound. As used in this specification, the term "percent" refers to a weight percent; it is the ratio of the weight of the component involved divided by the combined weights of all the components involved times 100. Thus, e.g., the term "percent", when applied to the cyclic nitrogen compound, refers to the weight of the cyclic nitrogen compound times 100 divided by the combined weight of the cyclic nitrogen compound, the phoshorus compound, the acid, and the water.

The cyclic nitrogen compound used in the intumescent coating composition of this invention is described by the formula

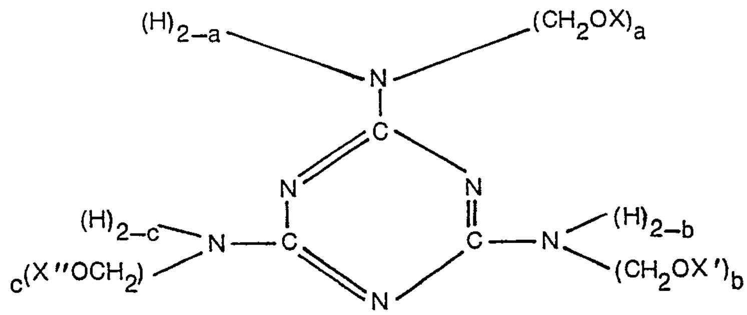

wherein a, b, and c are integers selected from the group consisting of 1 and 2, a plus b plus c equal about 3 to 6, and X, X', and X'' are independently selected from the group consisting of hydrogen and —$CH_3$. It is preferred that at least one of said X, X', and X'' groups be —$CH_3$. This cyclic nitrogen compound may be prepared by reacting at least three moles of formaldehyde per mole of melamine and then etherifying the methylol groups by reaction of the methylol melamine with methanol in the presence of an acid catalyst. Oligomers derived from said cyclic nitrogen compound also may be used in the intumescent composition of this invention.

In the more preferred cyclic nitrogen compound used in the intumescent composition of this invention, at least about two of said X, X', and X'' groups are —$CH_3$. In the most preferred embodiment, at least three of said X, X', and X'' groups are —$CH_3$.

It is preferred that from 35 to 50 percent (by weight) of said cyclic nitrogen compound be used in the intumescent composition of this invention. The trimethylated trimethylol melamine, tetramethylated tetramethylol melamine, pentamethylated pentamethylol melamine, and hexamethylated hexamethylol melamine compound are all well known to the art; and all of them may be used in the intumescent composition of this invention. Mixtures of said compounds may also be used in the intumescent composition of this invention.

The intumescent composition of this invention also contains a phosphorus compound. It is preferred that said composition contain from 10 to 50 percent (by weight) of a phosphorus compound; it is more preferred that it contain from 14 to 40 percent (by weight) of said phosphorus compound.

It is preferred that the phosphorus compound used in the intumescent composition of this invention be selected from the group consisting of

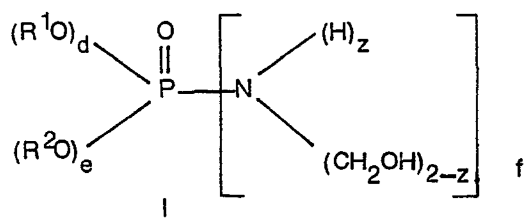

I

and

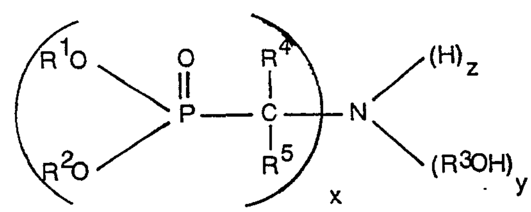

II

and mixtures thereof, wherein d is an integer of from 0 to 2, e is an integer of from 0 to 2, f is an integer of from 1 to 3, and d plus e plus f equal 3; z is an integer of 0 to 1; x is an integer of 1 to 2, y is an integer of 1 to 2, and x plus y plus z equal 3; $R^1$, $R^2$, and $R^3$ are selected from group consisting of alkyl radicals containing from 1 to 6 carbon atoms and haloalkyl radicals containing from 2 to 6 carbon atoms, provided that when $R^3$ is haloalkyl it contains at least 3 carbon atoms; $R^4$ and $R^5$ are selected from the group consisting of hydrogen, alkyl radicals, and haloalkyl radicals, wherein said radicals contain from 1 to 6 carbon atoms; and the total number of carbon atoms in the $R^1$, $R^2$, $R^3$, $R^4$, and $R^5$ groups does not exceed 14.

In a preferred embodiment, the phosphorus compound is soluble in either the water and/or the acid components of the composition; in this preferred embodiment, when one part of the phosphorus compound is mixed with no more than one part of the component in which it is soluble at 25 degrees centigrade, a one-phase solution is obtained.

The phosphorus compounds described by formula I may be made by methods well known to the art. Thus, for example, some may be prepared in accordance with the following reaction scheme:

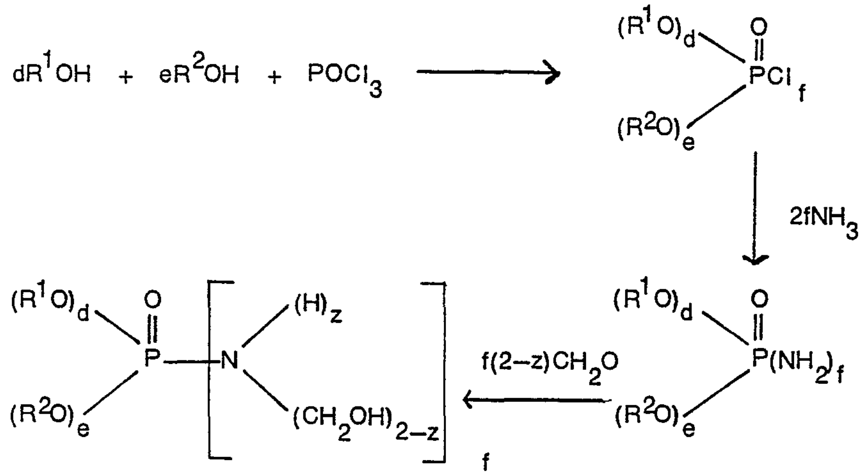


I

Some of the preferred phosphorus compounds which are described by formula I and can be utilized in the intumescent composition of this invention are, e.g., diethyl-N,N-dimethylol phosphoramidate, trichloroneopentyl-chloroethyl-N,N-dimethylol phosphoramidate, dimethyl-N,N-dimethylol phosphoramidate, methyl ethyl-N,N-dimethylol phosphoramidate, hexyl ethyl-N,N-dimethylol phosphoramidate, tribromoneopentyl methyl-N,N-dimethylol phosphoramidate, propylpentyl-N-methylol phosphoramidate, diethyl-N-methylol phosphoramidate, methyl propyl-N,N-dimethylol phosphoramidate, ethyl propyl-N-methylol phosphoramidate, dibutyl-N,N-dimethylol phosphoramidate, pentyl butyl-N,N-dimethylol phosphoramidate, dipropyl-N,N-dimethylol phosphoramidate, bis-(2-bromoethyl)-N,N-dimethylol phosphoramidate, chloroethyl-chloroneopentyl-N,N-dimethylol phosphoramidate, bis-(2-chloroethyl)-N,N-dimethylol phosphoramidate, bis-(2,3-dibromopropyl)N,N-dimethylol phosphoramidate, and the like. Mixtures of these compounds also may be utilized.

Other phosphorus compounds described by formula I which can be utilized in the intumescent composition of this invention include, e.g., hexamethylol phosphorotriamide, chloroneopentyl-N,N,N′,N′-tetramethylol phosphorodiamidate, chloroethyl-N,N′-dimethylol phosphorodiamidate, bis(N,N-dimethylol) phosphorodiamidate, propyl bis(N,N-dimethylol) phosphorodiamidate, and the like. Mixtures of these compounds also may be used.

The phosphorus compound described by formula II may be prepared by conventional methods well known to the art. Thus, the following reaction scheme may be used:

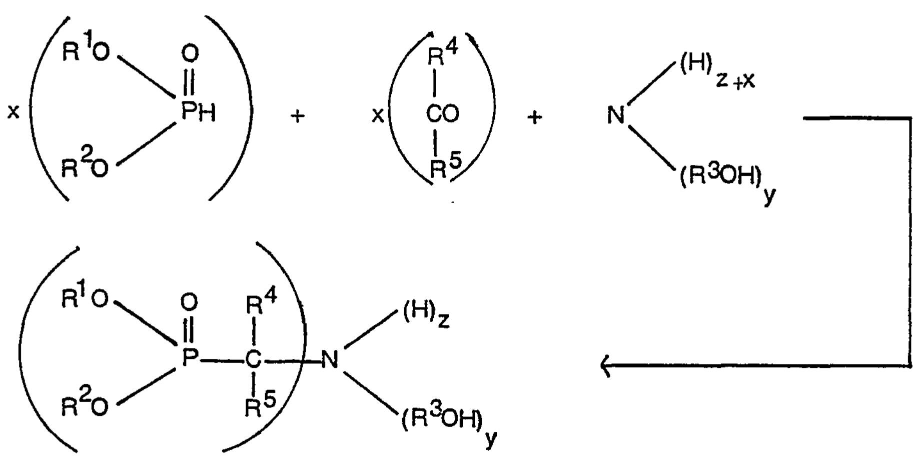

It is preferred that z be zero, and that $R^1$ and $R^2$ be independently selected from the group consisting of alkyl radicals containing from 1 to 4 carbon atoms and haloalkyl radicals containing from 2 to 4 carbon atoms, that $R^3$ be alkyl of from 1 to 4 carbon atoms, and that $R^4$ and $R^5$ be hydrogen. In a more preferred embodiment, x is 1, y is 2, and $R^1$ and $R^2$ are independently selected from the group consisting of ethyl and haloethyl.

When $R^1$ and $R^2$ is haloalkyl, the halogen substituent(s) is selected from the group consisting of bromine, chlorine, or mixtures thereof.

Some of the phosphorus compounds which are described by formula II and which can be utilized in the intumescent composition of this invention include, e.g., the phosphorus compounds described in

Table I. Mixtures of these compounds also may be utilized. Other comparable compounds will readily suggest themselves to those skilled in the art for use in the instumescent composition of this invention; they are also intended to be comprehended within the scope of this invention.

TABLE I

| $R_1$ | $R_2$ | $R_3$ | $R_4$ | $R_5$ | X | Y | Z |
|---|---|---|---|---|---|---|---|
| ethyl | ethyl | ethylene | H | H | 1 | 2 | 0 |
| chloroethyl | chloroethyl | ethylene | H | H | 1 | 2 | 0 |
| methyl | methyl | propylene | H | H | 1 | 2 | 0 |
| ethyl | butyl | ethylene | H | H | 1 | 2 | 0 |
| chloroiso-propyl | chloroiso-propyl | propylene | H | H | 1 | 2 | 0 |
| bromoethyl | bromomethyl | ethylene | H | H | 1 | 2 | 0 |
| bromopropyl | bromopropyl | propylene | H | H | 2 | 1 | 0 |
| ethyl | ethyl | ethylene | methyl | methyl | 1 | 2 | 0 |
| chloroethyl | chloroethyl | propylene | chloromethyl | chloromethyl | 1 | 2 | 0 |
| ethyl | ethyl | chloroiso-propylene | H | H | 1 | 1 | 1 |
| tribromoneo-pentyl | chloroethyl | ethylene | H | H | 1 | 2 | 0 |
| ethyl | ethyl | ethylene | H | H | 1 | 1 | 1 |

The intumescent composition of this invention contains an organo-phosphorus acid. It is preferred that said composition contain from 10 to 35 percent (by weight) of said acid; it is more preferred that said composition contain from 14 to 28 percent (by weight) of said acid. The acid used in the intumescent composition of this invention is selected from the group consisting of

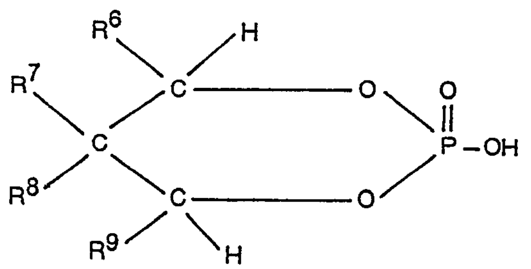 ,

III

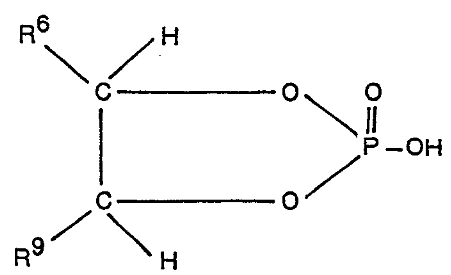 ,

IV

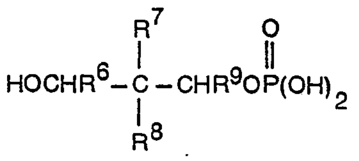 ,

V

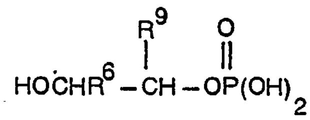 ,

VI

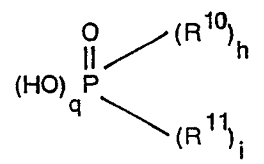 ,

VII

and mixtures thereof, wherein $R^6$, $R^7$, $R^8$ and $R^9$ are independently selected from the group consisting of hydrogen, alkyl of from 1 to 3 carbon atoms, haloalkyl of from 1 to 3 carbon atoms, and hydroxyalkyl of

from 1 to 3 carbon atoms, provided that the total number of carbon atoms in the $R^6$, $R^7$, $R^8$, and $R^9$ groups does not exceed 5; wherein q is an integer of from 1 to 2, h and i are integers independently selected from the group consisting of 0, 1, and 2, and q plus h plus i equals 3; and wherein $R^{10}$ and $R^{11}$ are independently selected from the group consisting of alkyl of from 1 to 6 carbon atoms, haloalkyl of from 1 to 6 carbon atoms and from 1 to 3 halogen atoms, hydroxy-polyalkyleneoxy containing 2 to 6 carbon atoms and 2 to 6 oxygen atoms, alkoxy of from 1 to 6 carbon atoms, hydroxyalkoxy of from 2 to 6 carbon atoms, haloalkoxy of from 2 to 6 carbon atoms and from 1 to 3 halogen atoms,

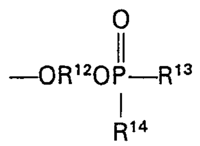

wherein $R^{12}$ is selected from the group consisting of alkylene of from 2 to 6carbon atoms, haloalkylene of from 3 to 6 carbon atoms and from 1 to 3 halogen atoms, polyalkyleneoxy containing 2 to 6 carbon atoms and 1 to 5 oxygen atoms, and hydroxyalkylene of from 3 to 6 carbon atoms and 1 to 4 hydroxyls, and $R^{13}$ and $R^{14}$ are independently selected from the group consisting of hydrogen, hydroxyl, alkyl of from 1 to 6 carbon atoms, haloalkyl of from 1 to 6 carbon atoms and from 1 to 3 halogen atoms, hydroxy-polyalkyleneoxy containing 2 to 6 carbon atoms and 2 to 6 oxygen atoms, alkoxy of from 1 to 6 carbon atoms, hydroxyalkoxy of from 2 to 6 carbon atoms, and haloalkoxy of from 2 to 6 carbon atoms and 1 to 3 halogen atoms, provided that the total number of carbon atoms in the $R^{10}$ and $R^{11}$ groups does not exceed 8.

Some of the acids described by formulae III and IV include, e.g., 2 - hydroxy - 2 - oxo - 1,3,2-dioxaphosphorinane, 2 - hydroxy - 1,3,2 - dioxaphospholane - 2 - oxide, 2 - hydroxy - 5 - methyl-2 - oxo - 1,3,2 - dioxaphosphorinane, 2 - hydroxy - 4 - methyl - 1,3,2 - dioxaphospholane - 2 - oxide, 2 - hydroxy - 4 - methyl - 2 - oxo - 1,3,2 - dioxaphosphorinane, 2 - hydroxy - 5 - ethyl - 2 - oxo - 1,3,2-dioxaphosphorinane, 2 - hydroxy - 4 - ethyl - 1,3,2 - dioxaphosphospholane - 2 - oxide, 2 - hydroxy - 4-ethyl - 2 - oxo - 1,3,2 - dioxaphosphorinane, 2 - hydroxy - 5 - n - propyl - 2 - oxo - 1,3,2 - dioxaphosphorinane, 2 - hydroxy - 4 - n - propyl - 1,3,2 - dioxaphospholane - 2 - oxide, 2 - hydroxy - 4 - n-propyl - 2 - oxo - 1,3,2 - dioxaphosphorinane, 2 - hydroxy - 5 - isopropyl - 2 - oxo - 1,3,2 - dioxaphosphorinane, 2 - hydroxy - 4 - isopropyl - 4 - methyl - 1,3,2 - dioxaphospholane - 2 - oxide, 2-hydroxy - 4 - isopropyl - 2 - oxo - 1,3,2 - dioxaphosphorinane, 2 - hydroxy - 5 - chloroethyl - 2 - oxo-1,3,2 - dioxaphosphorinane, 2 - hydroxy - 4- chloroethyl - 1,3,2 - dioxaphospholane - 2 - oxide, 2-hydroxy - 4 - chloroethyl - 2 - oxo - 1,3,2 - dioxaphosphorinane, 2 - hydroxy - 5 - bromomethyl - 2-oxo - 1,3,2 - dioxaphosphorinane, 2 - hydroxy - 4 - bromoethyl -1,3,2 - dioxaphospholane - 2 - oxide, 2 - hydroxy - 4 - bromoethyl - 2 - oxo - 1,3,2 - dioxaphosphorine, 2 - hydroxy - 5 - chloropropyl - 2-oxo - 1,3,2 - dioxaphosphorinane, 2 - hydroxy - 4 - chloropropyl - 1,3,2 - dioxaphospholane - 2 - oxide, 2 - hydroxy - 4 - chloropropyl - 2 - oxo - 1,3,2 - dioxaphosphorinane, 2 - hydroxy - 5 - bromopropyl - 2 - oxo - 1,3,2 - dioxaphosphorinane, 2 - hydroxy - 4 - bromopropyl - 1,3,2 - dioxaphospholane - 2 - oxide, 2 - hydroxy - 4 - bromopropyl - 2 - oxo - 1,3,2 - dioxaphosphorinane, 2-hydroxy - 5,5 - dimethyl - 2 - oxo - 1,3,2 - dioxaphosphorinane, 2 - hydroxy - 4,5 - dimethyl - 2 - oxo-1,3,2 - dioxaphospholane - 2 - oxide, 2 - hydroxy - 5,5 - bis(bromomethyl) - 2 - oxo - 1,3,2-dioxaphosphorinane, and the like.

Difunctional compounds of formulae V and VI may be utilized in the intumescent composition of this invention. These compounds usually contain both acid and hydroxy substituents. They may be used to replace either the acid compound and/or the phosphorus compound. Some of the difunctional compounds which may be utilized in the composition of this invention include, e.g., 2-hydroxyethyl phosphoric acid, 3-hydroxy-2-propyl phosphoric acid, 1-hydroxy-2-butyl phosphoric acid, 2-hydroxy-2,2-diethyl-1-propyl phosphoric acid, 2,2-dimethyl-3-hydroxy-1-propyl phosphoric acid, 3-hydroxy-1-propyl phosphoric acid, 2,2-bis(bromomethyl)-3-hydroxy-1-propyl phosphoric acid, 2,2-bis(chloromethyl)-3-hydroxy-1-propyl phosphoric acid, 2,2-bis(chloroethyl)-3-hydroxy-1-propyl phosphoric acid, 2,2-bis(hydroxymethyl)-3-bromo-1-propyl phosphoric acid, 2,2-bis(hydroxymethyl)-3-chloro-1-propyl phosphoric acid, and the like.

Some of the acids described by formula VII are shown in Table II.

TABLE II

| $R_{10}$ | $R_{11}$ | q | h | i |
|---|---|---|---|---|
| chloromethyl | | 2 | 1 | 0 |
| 2′-hydroxyethoxyethyl | | 2 | 1 | 0 |
| 2′-hydroxyethoxyethyl | | 1 | 2 | 0 |
| 2,2,2 tris-hydroxy methyl ethyl | | 2 | 1 | 0 |
| 4-hydroxy-1-butyl | | 2 | 1 | 0 |
| chloroethoxy | | 2 | 1 | 0 |
| chloroethoxy | | 1 | 2 | 0 |
| bromoethoxy | | 2 | 1 | 0 |
| bromoethoxy | | 1 | 2 | 0 |
| 1,3 dichloro-2-propoxy | | 2 | 1 | 0 |
| 1,3 dichloro-2-propoxy | | 1 | 2 | 0 |
| $—OCH_2CH_2OCH_2CH_2O\overset{O}{P}(OH)_2$ | | 2 | 1 | 0 |
| $—OCH_2CH_2O\overset{O}{\overset{\|}{P}}(OH)_2$ | | 2 | 1 | 0 |
| 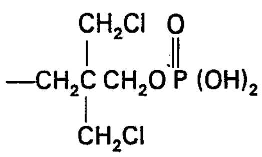 | | 2 | 1 | 0 |
| 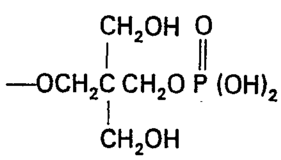 | | 2 | 1 | 0 |
| methyl | | 2 | 1 | 0 |
| methyl | | 1 | 2 | 0 |
| ethoxy | | 2 | 1 | 0 |
| ethoxy | | 1 | 2 | 0 |
| hydroxy polyethyleneoxy (1—3 repeat units) | | 1 | 2 | 0 |
| hydroxy polyethyleneoxy (1—3 repeat units) | | 2 | 1 | 0 |
| chloroethyl | | 2 | 1 | 0 |
| chloroethyl | | 1 | 2 | 0 |
| tribromoneopentoxy | | 2 | 1 | 0 |

TABLE II (Continued)

| $R_{10}$ | $R_{11}$ | q | h | i |
|---|---|---|---|---|
| trichloroneopentoxy | | 2 | 1 | 0 |
| chloroneopentoxy | | 2 | 1 | 0 |
| butyl | | 2 | 1 | 0 |
| butyl | | 1 | 2 | 0 |
| butoxy | | 2 | 1 | 0 |
| butoxy | | 1 | 2 | 0 |
| $-OCH_2CH_2O\overset{O}{P}(OCH_2CH_2OCH_2CH_2OH)_2$ | | 2 | 1 | 0 |
| ethyl | butyl | 1 | 1 | 1 |
| ethoxy | butoxy | 1 | 1 | 1 |
| chloroethoxy | 1,3-dichloro-2-propoxy | 1 | 1 | 1 |
| chloroneopentoxy | chloroethoxy | 1 | 1 | 1 |
| chloromethyl | chloroethoxy | 1 | 1 | 1 |
| bromopropoxy | chloroethoxy | 1 | 1 | 1 |

Compounds III and V may be prepared by hydrolyzing an acid chloride of III in accordance with the following reaction scheme:

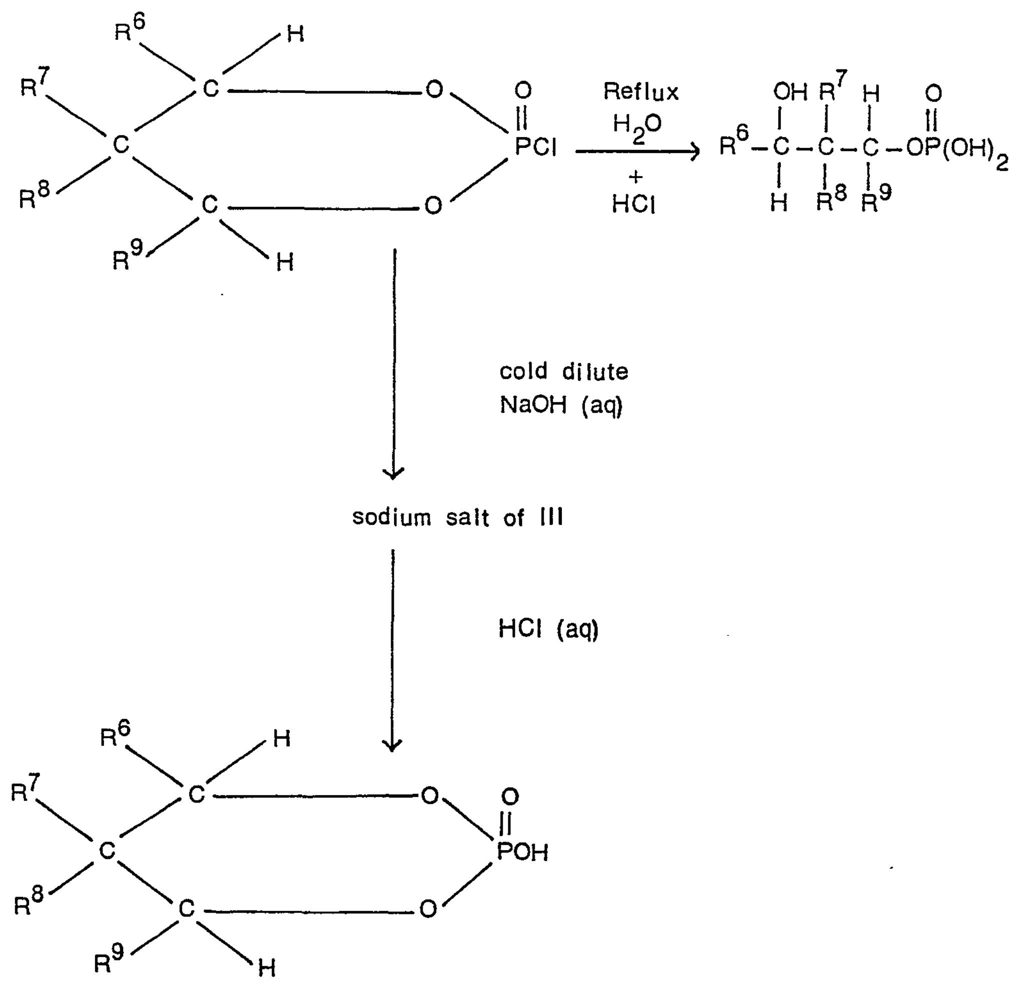


III

A comparable reaction scheme utilizing the acid chloride of structure IV as the reactant may be used to prepare compounds IV and VI.

Many of the acids described by formula VII may be prepared by hydrolyzing the acid chloride of compound VII. The acid chloride starting materials are well known to the art. When the acids are prepared by this method, the initial hydrolysis reaction may be conducted in water under mild conditions and/or in the presence of sufficient caustic so that the pH of the reaction mixture is at least 7.0. Hydrochloric acid may be used to neutralize the sodium salt of the acid and help release the free acid. The following reaction scheme may be used to prepare this compound.

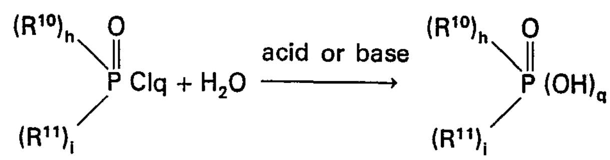


VII

It is preferred that the acid used in the intumescent composition of this invention be soluble in either water and/or the phosphorous compound used in this composition; when one part of the preferred acid is mixed with no more than one part of the component in which it is soluble at 25 degrees centigrade, a one phase solution is formed.

The intumescent composition of this invention contains water. It is preferred that said composition contain from 5 to 50 percent (by weight) of water. It is more preferred that said composition contain from 8 to 40 percent (by weight) of water.

The intumescent composition of this invention may be prepared by means known to the art. One may, e.g., mix in a dry state the cyclic nitrogen compound, the phosphorus compound, and the acid and then add the specified amount of water to the mixture. It is preferred, however, to mix solutions of said

compounds together; the concentrations of such solution are such that, after they are mixed together, the intumescent composition which results contains the specified amounts of water, cyclic nitrogen compound, phosphorus compound, and acid.

Generally, the components of the intumescent composition of this invention are mixed together for at least about 60 seconds, although longer and shorter mixing times may be used.

The intumescent composition of this invention should be applied to a wood substrate within about 240 minutes of the time it is prepared. Although it is useful for most wood substrates, it is especially useful for imparting durable flame retardancy to a plywood substrate.

Plywood is a material made by bonding wood together with an adhesive. The layers are usually veneer; they are generally no greater than about 0.1875" (0.4763 cm) thick for hardwood plywood and 0.1667" (0.4234 cm) thick for softwood plywood. The successive layers (plies) have their grains at a definite angle to each other, usually 90 degrees.

The composition of this invention may be advantageously utilized with any of the plywood substrates well known to the art. It may be applied to this type of substrate by any method known to the art such as, e.g., by spraying, brushing, or coating the composition onto the plywood. It is preferred that the dry solids add on of the intumescent composition of this invention be from 3 to 15 grams per square foot of plywood surface treated. The "add on" is determined by weighing the plywood substrate before and immediately after it is treated. The percent of the solids in the composition applied times this difference is the number of grams of dry solids applied; the number of grams of dry solids applied divided by the number of square feet treated is the "add on" referred to in this specification. One coat of the intumescent composition of this invention may be applied; it is preferred, however, to apply two or more coats.

After the composition of this invention is applied to the wood substrate, it is dried. It may be air dried, in which case up to about 30 hours should be allowed for it to dry. It may be dried by techniques well known to those skilled in the art. If heat is applied to the treated substrate, it is preferred to use a temperature of from 70 to 170 degrees centigrade for from 18 seconds to 20 minutes. It is more preferred to dry the treated substrate at a temperature of from 95 to 110 degrees centigrade for from 1 to 10 minutes.

The following examples illustrate the claimed invention. Unless otherwise specified, all parts are by weight, all temperatures are in degrees centigrade, all weights are expressed in grams, and all volumes are expressed in milliliters.

### Example 1

*N,N-dimethylol-bis(chloroethyl)phosphoramidate*

Thirteen hundred and forty-six grams (4.99 moles) of tris (chloroethyl phosphite) were charged to a three-liter flask equipped with a stirrer and a gas delivery tube. This reactant was cooled with an ice bath to a temperature of less than 20 degrees centigrade; and the addition of chlorine to the tris (chloroethyl phosphite) was commenced. The rate of chlorine addition was maintained so that the temperature of the reaction mixture did not rise above 25 degrees centigrade; chlorine was added to the reaction mixture over a period of 240 minutes until the chlorination reaction was complete (as was indicated by the reaction mixture turning to a yellow color). Dichloroethane by-product was removed from the reaction mixture by reduced pressure distillation. The reaction mixture was then maintained at ambient temperature and allowed to stand overnight.

Ammonia was added to the reaction mixture over a period of about 210 minutes; during the ammonia addition, chloroform was added to maintain the reaction mixture in a fluid state, and the reaction mixture was cooled so that the temperature of the reaction mixture did not exceed about 30 degrees centigrade. The reaction was continued until the ammonia had replaced all of the chlorine which was bonded directly to the phosphorus atom; the reaction was conducted over a period of 210 minutes.

Bis(chloroethyl)phosphoramidate was washed with water and filtered. A white solid with a melting point of 68 degrees centigrade was obtained; it contained 21.69 percent carbon, 4.66 percent hydrogen, 30.6 percent chlorine, 6.05 percent nitrogen, and 13.57 percent phosphorus.

N,N-dimethylol-bis(chloroethyl) phosphoramidate was prepared by dissolving bis(chloroethyl) phosphoramidate in a 37 percent aqueous solution of formaldehyde; about 2.1 moles of formaldehyde per mole of phosphoramidate were present in the reaction mixture. The pH of the reaction mixture was maintained at about 10 during the addition of the formaldehyde to the phosphoramidate; after these reactants formed a solution, however, the pH of the reaction mixture was adjusted to about 7 via the addition of dilute hydrochloric acid to the reaction mixture. The product obtained was dried and subjected to $H^1$ and $P^{31}$ N.M.R. analyses; the spectra obtained were substantially in accordance with those expected from the proposed structure.

### Example 2

*2,2-bis(bromomethyl)-3-hydroxy-1-propyl phosphoric acid*

Five thousand two hundred and forty grams (20.0 moles) of dibromoneopentyl glycol and 3,070 grams of phosphoryl chloride were placed in a 12-liter round bottom flask equipped with a stirrer,

heating mantle and condenser. The reaction mixture was consecutively maintained at ambient temperature for 30 minutes, a temperature of 40—45 degrees centigrade for 60 minutes, a temperature of 50 degrees centigrade for 60 minutes, a temperature of 70 degrees centigrade for 60 minutes, a temperature of 90 degrees centigrade for 120 minutes, and a temperature of 110 degrees centigrade (under aspirator vacuum) for 60 minutes. The reaction mixture was allowed to stand overnight. The reaction mixture thus formed was subjected to an absolute pressure of about 25 millimeters of mercury for about 360 minutes during which water and hydrogen chloride were removed from the mixture and the reaction temperature was allowed to increase to a maximum temperature of about 100 degrees centigrade.

The phosphoric acid obtained was a semi-solid material. The product was dissolved in sufficient water to produce a 60 percent solution. Elemental analysis indicated that it contained 27.0 percent of bromine; according to theory, it should have contained 28.5 percent of bromine.

Example 3

Fifty parts of an 80 percent aqueous solution of Aerotex Resin M—3®, a methylated trimethylol melamine compound available from the American Cyanamide Corporation, were mixed with 25 parts of a 60 percent aqueous solution of N,N-dimethylol-bis(chloroethyl) phosphoramidate and 25 parts of a 60 percent aqueous solution of 2,2-bis(bromomethyl)-3-hydroxy-1-propyl phosphoric acid; the former compound was prepared in substantial accordance with the procedure described in Example 1, and the latter compound was prepared in substantial accordance with the procedure described in Example 2.

Two coats of this formulation were brushed onto a lauan plywood sample (which was 24.0" (60.96 cm) long, 3.5" (8.89 cm) wide, and 0.1875" (0.4763 cm) thick) to a dry solids add on of 8 grams per square foot (86.11 gms per sq metre). The coated sample was then dried at a temperature of about 100 degrees centigrade for about 5 minutes. Thereafter the sample was subjected to a two-foot (60.96 cm) tunnel test to determine the flame spread rate; this test was conducted in substantial accordance with the procedure described in an article entitled "Two-Foot Tunnel Test", Journal of Paint Technology, Vol. 11, No. 565, February 1972, pp 43—47; however the panels were not aged as described in this article.

The two-foot tunnel test is similar to the UL Steiner 25-foot tunnel test described by ASTM E84—68. In the former test, the two-foot tunnel was inclined 28 degrees from the horizontal and utilized approximately 96 square inches (619.35 sq cm) of test surface. The test specimen was mounted on an angle iron frame in such a way that the surface to be evaluated formed the ceiling of the tunnel. A standard meeker burner was placed at the lower end of the tunnel, and the specimen was subjected to the flame from this burner for five minutes; during the first four minutes, the length of the advance of the flame front up the inclined panel was recorded at 15 second intervals. The flame lengths were measured by observing the flame front advance through a calibrated window located on the side of the tunnel. The tunnel was calibrated prior to specimen testing by determining the difference in flame lengths of a specimen of asbestos cement board and a specimen of red oak; this difference, by introduction of a constant K, was given a flame spread rating ("FRS") of 100. The flame spread rate calculation was made in accordance with the formula F.S.R. = $(L_n—L_a)K$ wherein F.S.R. was the flame spread rating, $L_n$ was the observed flame of the specimen tested, $L_a$ was the flame for asbestos cement board, $L_o$ was the observed flame length for the red oak sample, and

$$K = \frac{100}{L_o - L_a}.$$

The samples were weighed both before and after being tested in the two-foot (60.96 cm) tunnel, and the percent weight loss due to combustion of the sample was determined.

The coated plywood sample of this example had a flame spread rating of 20 and lost about 6 percent of its weight after the two foot tunnel test. The coating intumesced during the test and protected the wood surface.

A control experiment was conducted wherein the lauan plywood sample which was subjected to the two foot tunnel test was not coated with the intumescent composition of this invention. The uncoated plywood sample had a flame spread rating of 95 and lost about 18 percent of its weight after the two foot tunnel test.

Examples 4—16

In substantial accordance with the procedure described in Example 3, various coating formulations were prepared, applied to lauan plywood samples measuring 24" x 3.5" x 0.1875" (60.96 cm x 8.89 cm x 0.4763 cm), and dried; and the coated plywood samples were tested in the two foot (60.96 cm) tunnel to determine their flame spread ratings and how much weight they lost during this test. Table III presents the results of these experiments, indicating the formulations and conditions used in each Example.

TABLE III

| Example Number | 4 | 5 | 6 | 7 | 8 | 9 | 10 | 11 | 12 | 13 | 14 | 15* | 16* |
|---|---|---|---|---|---|---|---|---|---|---|---|---|---|
| Parts of 80 percent aqueous solution of methylated trimethylol melamine | 40 | 40 | 40 | 60 | 70 | 0 | 50 | 50 | 0 | 0 | 50 | 50 | 50 |
| Parts of methylated hexamethylol melamine | 0 | 0 | 0 | 0 | 0 | 50 | 0 | 0 | 40 | 50 | 0 | 0 | 0 |
| Parts of 60 percent aqueous solution of N,N-dimethylol-bis(chloroethyl)phosphoramidate | 30 | 40 | 20 | 20 | 15 | 0 | 0 | 0 | 0 | 0 | 0 | 0 | 0 |
| Parts of 60 percent aqueous solution of 2,2-bis(bromomethyl)-3-hydroxy-1-propyl phosphoric acid | 30 | 20 | 40 | 20 | 15 | 25 | 7 | 25 | 25 | 25 | 25 | 0 | 0 |
| Parts of diethyl-N,N-bis (2-hydroxyethyl)amino methane phosphonate | 0 | 0 | 0 | 0 | 0 | 25 | 25 | 25 | 25 | 25 | 25 | 25 | 25 |
| Parts of water | 0 | 0 | 0 | 0 | 0 | 0 | 13 | 0 | 10 | 0 | 0 | 10 | 0 |
| Dry solids add on (Grams/square foot) | 7 | 8 | 8 | 6 | 5 | 7 | 8 | 8 | 7 | 7 | 8 | 12 | 8 |
| (Grams/square metre) | 75.35 | 86.11 | 86.11 | 64.58 | 53.82 | 75.35 | 86.11 | 86.11 | 75.35 | 75.35 | 86.11 | 129.17 | 86.11 |
| Weight loss, percent | 6 | 6 | 5 | 6 | 6 | 5 | ND | 5 | 5 | 5 | 5 | 6 | 6 |
| Flame spread rating | 20 | 20 | 20 | 20 | 30 | 20 | 35 | 20 | 30 | 30 | 30 | 35 | 30 |

*Formulation also contained 15 parts of dibutyl hydrogen phosphate

**Formulation also contained 25 parts of a 60 percent aqueous solution of mono-(tribromoneopentyl) phosphoric acid

Examples 17—18

In substantial accordance with the procedure described in Example 3, a coating formulation containing 50 parts of a 80 percent aqueous solution of methylated trimethylol melamine, 25 parts of a 60 percent aqueous solution of N,N-dimethylol-bis(chloroethyl)phosphoramidate, and 25 parts of a 60 percent aqueous solution of 2,2,-bis(bromomethyl)-3-hydroxy-1-propyl phosphoric acid was prepared, coated onto a lauan plywood sample measuring 24.0" x 3.5" x 0.1875" (60.96 cm x 8.89 cm x 0.4763 cm) to a dry solids add on of 7 grams per square foot (75.35 gms per sq metre), dried, and tested to determine how much smoke and carbon monoxide the coated plywood sample generated during pyrolysis.

In accordance with National Fire Protection Association Test number 258, the coated plywood sample was tested under non-flaming conditions in the NBS Smoke Chamber. This test is described in an article by D. Gross, J. Loftus, and A. F. Robertson entitled "Method for Measuring Smoke from Burning Materials", *Symposium on Fire Test Methods—Restraint & Smoke,* 1966, ASTM STP 422, Am. Soc. Testing Mats., 1967, p. 166; this article is hereby incorporated by reference into this specification.

Both a plywood sample coated with the formulation of this Example and an uncoated control sample of the lauan plywood sample were tested. The maximum smoke density ("Dmc") generated by the plywood sample and the time to reach "Dmc" in minutes during the test was determined, as was the weight loss of the plywood sample (percent weight loss). The maximum smoke density was corrected for smoke deposited on the optical windows. Air samples were continually removed from the chamber at the rate of 0.5 liter/minute. The amount of carbon monoxide present in these samples was determined (and was expressed as "CO conc., ppm"). The results of these experiments are shown in Table IV.

TABLE IV

| Example Number | Coating Description | Dmc | Percent Weight Loss | Time to 2Dmc (Minutes) | CO Conc. (p.p.m.) 5 min. | 10 min. | 20 min. |
|---|---|---|---|---|---|---|---|
| 17 | None | 324 | 82 | 9.5 | 420 | 1600 | 3880 |
| 18 | Coating of Example 3 | 152 | 36 | 20. | 60 | 190 | 570 |

Example 19

A coating formulation containing 50 parts of an 80 percent aqueous solution of methylated trimethylol melamine, 25 parts of a 60 percent aqueous solution of 2,2-bis(bromomethyl)-3-hydroxy-1-propyl phosphoric acid, and 25 parts of "Fyrol 6" (a commercial flame retardant available from the Stauffer Chemical Corporation which contains diethyl-N,N-bis(2-hydroxyethyl)amino methane phosphonate) was prepared. In substantial accordance with the procedures described in Examples 3 and 20—21, this coating was applied to a lauan plywood sample measuring 24.0" x 3.5" x 0.1875" (60.96 cm x 8.89 cm x 0.4763 cm) to a dry solids add on of 7 grams per square foot (75.35 gms per square metre), dried, and tested in the NBS chamber to determine how much smoke and carbon monoxide the coated plywood sample generated during pyrolysis.

The maximum smoke density of the coated sample of this Example was 180: the time to Dmc was 20 minutes; and the sample lost 41 percent of its weight during the test. The carbon monoxide concentrations in the air samples at 5 minutes, 10 minutes, and 20 minutes after the test began were 60, 200, and 670 parts per million, respectively.

Example 20

A coating formulation containing 50 parts of an 80 percent aqueous solution of methylated hexamethylol melamine (available as "Resimene 745"® from the Monsanto Chemical Corporation); 25 parts of a 60 percent aqueous solution of 2,2-bis(bromomethyl)-3-hydroxy-1-propyl phosphoric acid and 25 parts of diethyl-N,N-bis(2-hydroxyethyl)amino methane phosphanate was prepared. In substantial accordance with the procedures described in Examples 3 and 17—19, this coating was applied to a lauan plywood sample measuring 24.0" x 3.5" x 0.1875" (60.96 cm x 8.89 cm x 0.4763 cm) to a dry solids add on of 7 grams per square foot (75.35 gms per sq. metre), dried, and tested in the NBS chamber to determine how much smoke and carbon monoxide the coated plywood sample generated.

The maximum smoke density of the coated sample of this Example was 130. The time to "Dmc" was 20 minutes, and the sample lost 32 percent of its weight during the test. The carbon monoxide

concentrations in the air samples at 5 minutes, 10 minutes, and 20 minutes after the test began were 40, 130, and 430 parts per million, respectively.

Example 21

Fifty parts of an 80 percent aqueous solution of methylated trimethylol melamine were mixed with 25 parts of a 60 percent aqueous solution of pentaerythritol disphosphoric acid and 25 parts of diethyl-N,N-bis(2-hydroxyethyl)amino methane phosphonate; in accordance with the procedure described in Example 3, the coating formulation so obtained was applied to a lauan plywood sample measuring 24.0" x 3.5" x 0.1875" (60.96 cm x 8.89 cm x 0.4736 cm) to a dry solids add one of 10 grams per square foot (107.64 gms per sq. metre) and dried; and the coated plywood sample was then tested in the two-foot (60.96 cm) tunnel test.

The coated plywood sample of this Example had a flame spread rating of 30 and lost about 6 percent of its weight during the two foot (60.96 cm) tunnel test.

Example 22

A coating containing 50 parts of an 80 percent aqueous solution of methylated hexamethylol melamine, 25 parts of diethyl N,N-bis(hydroxyethyl)amino methane phosphonate, and 25 parts of a 60 percent aqueous solution of monotribromoneopentyl phosphoric acid was prepared, applied to a lauan plywood sample measuring 24.0" x 3.5" x 0.1875" (60.96 cm x 8.89 cm x 0.4763 cm) to a dry solids add on of 7 grams per square foot (75.35 gms per sq metre), and dried. The coating plywood sample was subjected to the two foot (60.96 cm) tunnel test described in Example 3; it had a flame spread rating of 20 and lost 6 percent of its weight during the test.

Comparative Examples 23—28

Various compositions which are outside the scope of the claims of this application were evaluated. In each of these examples, the composition described was coated onto a lauan plywood sample measuring 24.0" x 3.5" x 0.1875" (60.96 cm x 8.89 cm x 0.4763 cm) to the dry solids add on indicated, and the coated plywood sample was dried and tested in the two foot (60.96 cm) tunnel in accordance with the procedure described in Example 3. Table V indicates what occurred in these experiments.

TABLE V

| Example Number | Composition Tested | Dry Solids Add On gm/sq ft | Dry Solids Add On gm/sq metre | Flame Spread Rating | Percent Weight Loss |
|---|---|---|---|---|---|
| 23 | 50 parts 80% aqueous soln. methylated trimethylol melamine* 50 parts 60% aqueous soln. N,N-bismethylol-bis(chloroethyl) phosphoramidate | 8 | 86.11 | 70 | 13 |
| 24 | 50 parts of methylated hexamethylol melamine 48 parts diethyl-N,N-bis(2-hydroxyethyl)amino methane phosphonate 2 parts paratoluene sulfonic acid | 9 | 96.87 | 100 | 12 |
| 25 | 50 parts of methylated hexamethylol melamine 50 parts of diethyl-N,N-bis (2-hydroxyethyl)amino methane phosphonate | 8 | 86.11 | 115 | 14 |
| 26 | 50 parts 80% aqueous soln. methylated trimethylol melamine 25 parts diethyl-N,N-bis (2-hydroxyethyl)amino methane phosphonate 25 parts of the ammonium salt of dibutyl hydrogen phosphate | 7.5 | 80.73 | 70 | 12 |
| 27 | 50 parts 80% aqueous soln. methylated trimethylol melamine 25 parts diethyl-N,N-bis (2-hydroxyethyl)amino methane phosphonate 15 parts neopentyl glycol 10 parts water | 9 | 96.87 | 80 | 10 |
| 28 | 50 parts 80% aqueous soln. methylated trimethylol melamine 25 parts diethyl-N,N-bis (2-hydroxyethyl)amino methane phosphonate 25 parts of Antiblaze 19®** | 8 | 86.11 | 80 | N.D. |

*Coating tacky and does not cure well.

**Antiblaze 19® is a commercially available phosphorus-containing flame retardant which is sold by the Mobile Chemical Company.

Example 29

Fifty parts of an 80 percent aqueous solution of Aerotex Resin M—3® were mixed with 25 parts of a 60 percent aqueous solution of N,N-dimethylol-bis(chloroethyl)phosphoramidate and 25 parts of a 60 percent aqueous solution of 2,2-bis(bromomethyl)-3-hydroxy-1-propyl phosphoric acid. Two coats of this formulation were applied to samples of lauan plywood which were 24.0″ long × 3.5″ wide × 0.1875″ thick (60.96 cm × 8.89 cm × 0.4763 cm) to a dry solids add on of 9 grams per square foot (96.87 gms per sq metre); one side of each sample was coated, and the coated samples were then dried at a temperature of about 100 degrees centigrade for about 5 minutes.

One of the samples, the "control", was subjected to the two foot tunnel test described in Example 3 to determine its flame spread rating and the amount of weight it lost during the test. The other sample was subjected to accelerated aging conditions to determine whether the flame retardant properties of the coating of this invention were affected by heat, water, and ultra-violet light. In accordance with A.S.T.M. test G—26—70, the sample was tested in a "Weather-Ometer" (Model 25/18—WR manufactured by the Atlas Electric Device Company of Chicago, Illinois); the sample was maintained at a relative humidity of from about 60 to about 65, and a 120 minute cycle wherein there were 102 minutes of light followed by 18 minutes of light and spray was used. After being subjected to these conditions in the "Weather-Ometer" for 100 hours, the sample was tested in the two foot tunnel to determine its flame spread rating and the amount of weight it lost during the test.

The control sample, which was not subjected to UV exposure, had a flame spread rating of 30 and lost 5 percent of its weight. The sample which had been subjected to 100 hours of exposure conditions had a flame spread rating of 35 and about 5 percent of its weight was lost.

## Example 30

Fifty parts of an 80 percent aqueous solution of Aerotex Resin M—3® were mixed with 25 parts of diethyl-N,N-bis(2-hydroxyethyl)amino methane phosphonate and 25 parts of a 60 percent aqueous solution of 2,2-bis(bromomethyl)-3-hydroxy-1-propyl phosphoric acid. In accordance with the procedure of Example 3, two coats of this formulation were applied to both sides of samples of lauan plywood which were 24.0" long x 3.5" wide x 0.1875" thick (60.96 cm x 8.89 cm x 0.4763 cm) to a dry solids add on of 9 grams per square foot (96.87 gms per sq metre) on each side and the coated samples were then dried at a temperature of about 100 degrees centigrade for about 5 minutes.

One of the samples, the "control", was subjected to the two foot tunnel test described in Example 3. In accordance with the procedure described in Example 29, the other sample was subjected to accelerated aging conditions for 18 hours; the relative humidity was maintained at 60—65%, and a 120 minute cycle wherein there were 102 minutes of light followed by 18 minutes of light and spray was used. The sample subjected to these conditions was removed from the "Weather Ometer" after 18 hours and subjected to the two foot (60.96 cm) tunnel test.

The control sample had a flame spread rating of 30 and lost 5 percent of its weight during the two foot tunnel test. The sample which had been subjected to 18 hours of accelerated aging conditions had a flame spread rating of 30 and lost 5 percent of its weight during the two foot (60.96 cm) tunnel test.

## Comparative Examples 31—34

In accordance with the procedures described in Example 29 and 30, various formulations outside the scope of this invention were tested in the two foot tunnel to determine their flame spread ratings and the percent of weight they lost during the two foot tunnel test. Each formulation was applied to a dry solids add on of 9 grams per square foot (96.87 gms per sq metre) to samples of lauan plywood which were 24.0" long x 3.5" wide x 0.1875" thick (60.96 cm x 8.89 cm x 0.4763 cm); both sides of the lauan plywood samples were coated with the formulations. The coated plywood samples were dried at a temperature of about 100 degrees centigrade for about 5 minutes.

The two-foot (60.96 cm) tunnel tests were conducted for two plywood samples coated with each formulation—one on a sample which had not been subjected to accelerated aging conditions, and one on a sample which was subjected to the accelerated aging conditions described in Example 32 for 100 hours.

The formulation used in Example 31 contained 50 parts of an 80% aqueous solution of Aerotex Resin M—3® and 50 parts of a 60 percent aqueous solution of 2,2-bis(bromomethyl)-3-hydroxy-1-propyl phosphoric acid. The formulation used in Examples 32 contained 70 parts of an 80 percent aqueous solution of Aerotex Resin M—3®, 15 parts of an 85 percent aqueous solution of phosphoric acid, and 15 parts of water. The formulation used in Example 33 contained 50 parts of Aerotex Resin M—3® and 50 parts of diethyl-N,N-bis(2-hydroxyethyl) amino methane phosphonate. No formulation was used in Example 34—the uncoated plywood samples were tested at "time zero" and after 100 hours of accelerated aging.

The results of these experiments are presented in Table VI.

TABLE VI

| | Example No. | | | |
|---|---|---|---|---|
| | 31 | 32 | 33 | 34 |
| Flame Spread Rating, 0 Hours | 50 | 110 | 65 | 95 |
| Flame Spread Rating, 100 Hours | 50 | 110 | 85 | 115 |
| Percent Weight Loss, 0 Hours | 7 | 9 | 8 | 14 |
| Percent Weight Loss, 100 Hours | 7 | 12 | 8 | 15 |

The plywood sample of Example 32 generated black smoke during two foot (60.96 cm) tunnel tests of both the exposed and unexposed samples.

Examples 33—36

Other organo-phosphorus acids may be used in the intumescent composition of this invention with equally good results. Various formulations were prepared and tested in accordance with the procedure described in Example 3.

A formulation containing 50 parts of an 80 percent aqueous solution of methylated trimethylol melamine, 25 parts of diethyl-N,N-bis(2-hydroxyethyl)amino methane phosphonate, and 25 parts of 2-hydroxy-5,5-dimethyl-1,3,2-dioxo-phosphorinane, when applied to a dry solids add on of 8 grams per square foot, had a flame spread rating of 30 and lost 5 percent of its weight.

A formulation containing 25 grams of a 60 percent aqueous solution of bis(dichloroisopropyl)-phosphoric acid, 25 parts of diethyl-N,N-bis(2-hydroxyethyl)amino methane phosphonate, and 50 parts of an 80 percent aqueous solution of methylated trimethylol melamine, when applied to a dry solids add on of 10 grams per square foot, had a flame spread rating of 30 and a percent weight loss of about 5.5%.

A formulation containing 50 grams of an 80 percent aqueous solution of methylated trimethylol melamine, 25 parts of a 60 percent aqueous solution of bis(2-chloroethyl)phosphoric acid, and 25 parts of diethyl-N,N-bis(2-hydroxyethyl)amino methane phosphonate, when applied to a dry solids add on of 10 grams per square foot, had a flame spread rating of 35 and a percent weight loss of 6.

A formulation containing 10 parts of chloroneopentyl phosphoric acid, 55 parts of an 80 percent aqueous solution of methylated trimethylol melamine, 10 parts of water, and 25 parts of diethyl-N,N-bis(2-hydroxyethyl)amino methane phosphonate, when applied to a dry solids add on of 10 grams per square foot, had a flame spread rating of 35 and a percent weight loss of 6.

Examples 37—38

Some organo-phosphorous acids outside the scope of the claims of this case were evaluated in intumescent compositions; these acids contained aryl substituents. Various formulations were prepared and tested in accordance with the procedure of Example 3.

Formulations containing 50 parts of an 80 percent aqueous solution of methylated trimethylol melamine, 25 parts of diethyl-N,N-bis(2-hydroxyethyl)amino methane phosphonate, and an aryl-substituted organo-phosphor acid were prepared. The formulation of Example 37 contained 25 parts of a 60 percent aqueous solution of phenyl phosphonic acid. The formulation of Example 38 contained 25 parts of a 60 percent aqueous solution of phenyl phosphoric acid. Both formulations were applied to and add on of 10 grams per square foot.

The formulation of Example 37 had a flame spread rating of 110 and lost 14 percent of its weight. The formulation of Example 38 had a flame spread rating of 85 and lost 11 percent of its weight.

The above examples have been described for the purpose of illustration, not limitation. Many other modifications will suggest themselves to those skilled in the art; they are intended to be comprehended within the scope of the invention.

**Claims**

1. An intumescent composition containing a cyclic nitrogen compound, a hydroxy-containing organo-phosphorus compound, an organo-phosphorus acid, and water, wherein:

a) said cyclic nitrogen compound is

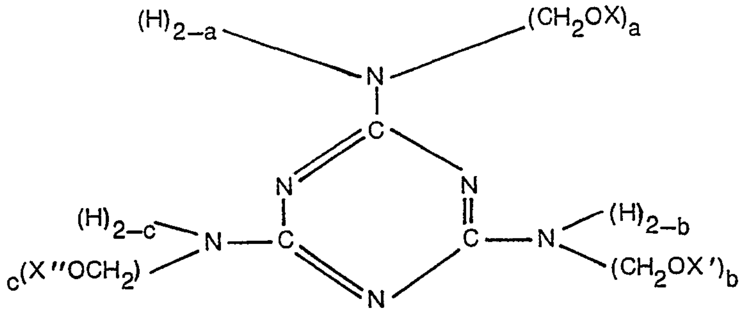

wherein a, b, and c are integers selected from the group consisting of 1 and 2, a plus b plus c equal 3 to 6, and X, X′, and X″ are independently selected from the group consisting of hydrogen and $—CH_3$;

b) said hydroxy-containing organo-phosphorus compound is selected from the group consisting of

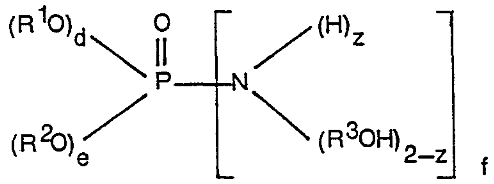

I

and

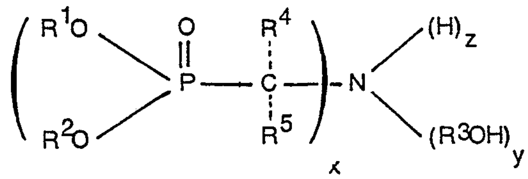

II

and mixtures thereof, wherein d is an integer of from 0 to 2, e is an integer of from 0 to 2, f is an integer of from 1 to 3, and d plus e plus f equal 3; z is an integer of 0 to 1; x is an integer of 1 to 2, y is an integer of 1 to 2, and x plus y plus z equal 3; $R^1$, $R^2$, and $R^3$ are selected from the group consisting of alkyl radicals containing from 1 to 6 carbon atoms and haloalkyl radicals containing from 2 to 6 carbon atoms, provided that when $R^3$ is haloalkyl it contains at least 3 carbon atoms; $R^4$ and $R^5$ are selected from the group consisting of hydrogen, alkyl radicals, and haloalkyl radicals, wherein said radicals contain from 1 to 6 carbon atoms; and the total number of carbon atoms in the $R^1$, $R^2$, $R^3$, $R^4$, and $R^5$ groups does not exceed 14; and

c) said acid is selected from the group consisting of

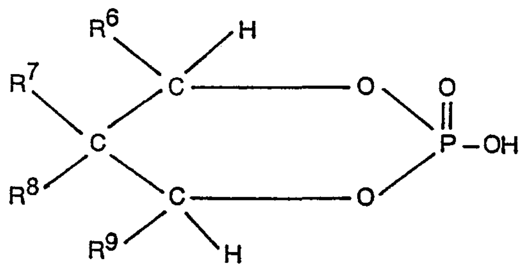

,

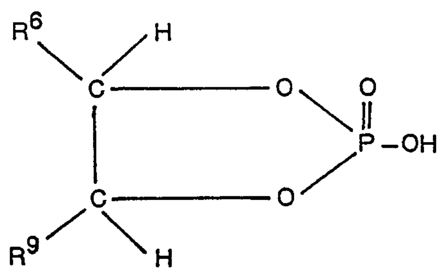

,

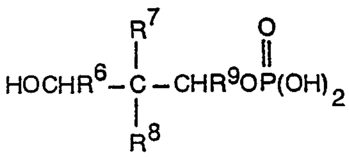

,

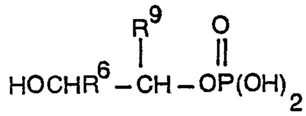

,

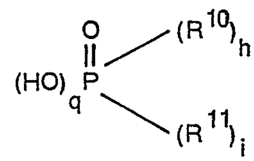

,

and mixtures thereof, wherein $R^6$, $R^7$, $R^8$ and $R^9$ are independently selected from the group consisting of hydrogen, alkyl of from 1 to 3 carbon atoms, haloalkyl of from 1 to 3 carbon atoms, and hydroxyalkyl of from 1 to 3 carbon atoms, provided that the total number of carbon atoms in the $R^6$, $R^7$, $R^8$, and $R^9$ groups does not exceed 5; wherein q is an integer of from 1 to 2, h and i are integers independently selected from the group consisting of 0, 1, and 2, and q plus h plus i equals 3; and wherein $R^{10}$ and $R^{11}$ are independently selected from the group consisting of alkyl of from 1 to 6 carbon atoms, haloalkyl of from 1 to 6 carbon atoms and from 1 to 3 halogen atoms, hydroxy-polyalkyleneoxy containing 2 to 6 carbon atoms and 2 to 6 oxygen atoms, alkoxy of from 1 to 6 carbon atoms, hydroxyalkoxy of from 2 to 6 carbon atoms, haloalkoxy of from 2 to 6 carbon atoms and from 1 to 3 halogen atoms,

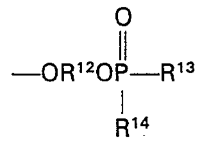

wherein $R^{12}$ is selected from the group consisting of alkylene of from 2 to 6 carbon atoms, haloalkylene of from 3 to 6 carbon atoms and from 1 to 3 halogen atoms, polyalkyleneoxy containing 2 to 6 carbon atoms and 1 to 5 oxygen atoms, and hydroxyalkylene of from 3 to 6 carbon atoms and 1 to 4 hydroxyls, and $R^{13}$ and $R^{14}$ are independently selected from the group consisting of hydrogen, hydroxyl, alkyl of from 1 to 6 carbon atoms, haloalkyl of from 1 to 6 carbon atoms and from 1 to 3 halogen atoms, hydroxy-polyalkyleneoxy containing 2 to 6 carbon atoms and 2 to 6 oxygen atoms, alkoxy of from 1 to 6 carbon atoms, hydroxyalkoxy of from 2 to 6 carbon atoms, and haloalkoxy of from 2 to 6 carbon atoms and 1 to 3 halogen atoms, provided that the total number of carbon atoms in the $R^{10}$ and $R^{11}$ groups does not exceed 8.

2. An intumescent composition as claimed in claim 1, wherein said composition contains from 10 to 50 percent (by weight) of said hydroxy-containing organo-phosphorus compound, from 10 to 35 percent (by weight) of said organo-phosphorus acid, from 30 to 60 percent (by weight) of said cyclic nitrogen compound, and from 5 to 50 percent (by weight) of water.

3. An intumescent composition as claimed in claim 2, wherein said composition contains from 14 to 40 percent (by weight) of said hydoxy-containing organo-phosphorus compound, from 14 to 28 percent (by weight) of said organo-phosphorus acid, and from 8 to 40 percent (by weight) of water.

4. An intumescent composition as claimed in claim 1, 2 or 3, wherein the total number of carbon atoms in said $R^1$, $R^2$, $R^3$, $R^4$, and $R^5$ groups does not exceed 10.

5. An intumescent composition as claimed in any one of the preceding claims, wherein said phosphorus compound is

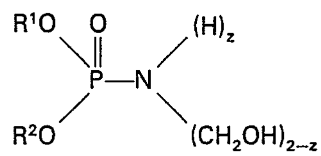

6. An intumescent composition as claimed in any one of claims 1 to 4, wherein said phosphorus compound is

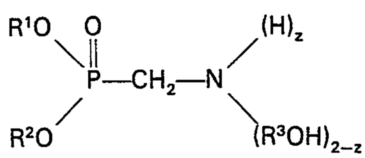

7. An intumescent composition as claimed in any one of the preceding claims, wherein z is 0.

8. An intumescent composition as claimed in any one of the preceding claims, wherein $R^1$ and $R^2$ are independently selected from the group consisting of alkyl radicals containing from 1 to 4 carbon atoms and haloalkyl radicals containing from 2 to 4 carbon atoms.

9. An intumescent composition as claimed in claim 8, wherein $R^1$ and $R^2$ are independently selected from the group consisting of ethyl and haloethyl.

10. An intumescent composition as claimed in claim 9, wherein $R^1$ and $R^2$ are chloroethyl.

11. An intumescent composition as claimed in claim 9, wherein $R^1$ and $R^2$ are ethyl.

12. An intumescent composition as claimed in any one of the preceding claims, wherein said organo-phosphorus acid is selected from the group consisting of 2,2-bis(bromomethyl)-3-hydroxy-1-propyl phosphoric acid, dibuty hydrogen phosphate, mono-(tribromoneopentyl) phosphoric acid, 2-hydroxy-5,5-dimethyl-1,3,2-dioxophosphorinane, bis(dichloroisopropyl)phosphoric acid, bis(2-chloroethyl) phosphoric acid, chloroneopentyl phosphoric acid, and mixtures thereof.

13. An intumescent composition as claimed in any one of the preceding claims, wherein a plus b plus c is 3.

14. An intumescent composition as claimed in claim 13, wherein a plus b plus c is 6.

15. An intumescent composition as claimed in any one of the preceding claims, wherein X, X′ and X″ are $CH_3$.

**Revendications**

1. Composition intumescente contenant un composé azoté cyclique, un composé organophosphoré contenant un ou plusieurs groupes hydroxy, un acide organophosphoré et de l'eau, où

a) le composé azoté cyclique est:

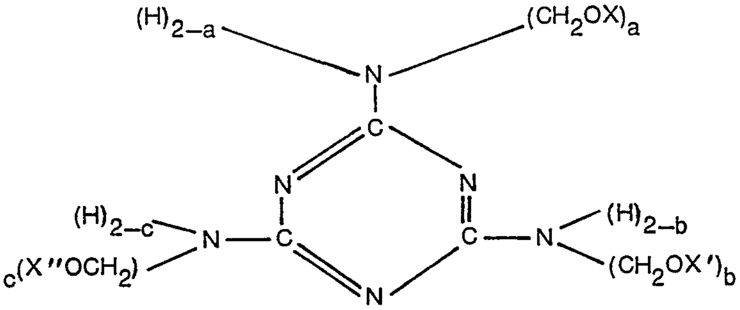

où a, b et c sont des nombres entiers choisis dans le groupe se composant de 1 et 2, a plus b plus c est égal à 3 à 6 et X, X′ et X″ sont indépendamment choisis dans le groupe se composant d'hydrogène et de $-CH_3$;

b) le composé organophosphoré contenant un ou plusieurs groupes hydroxy est choisi dans le groupe se composant de:

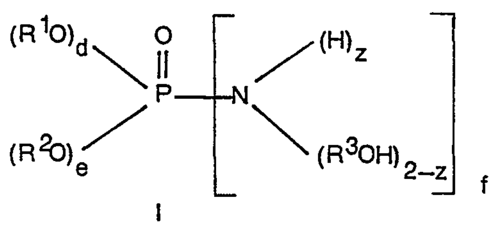

I

et

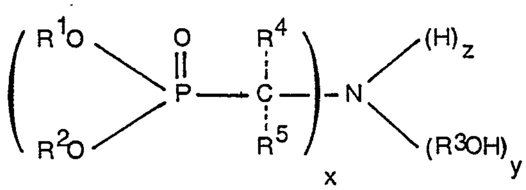

II

et de leurs mélanges, ou de est un nombre entier de 0 à 2, e est un nombre entier de 0 à 2, f est un nombre entier de 1 à 3 et d plus e plus f est égal à 3; z est un nombre entier de 0 à 1, x est un nombre entier de 1 à 2, y est un nombre entier de 1 à 2, et x plus y plus z est égal à 3; $R^1$, $R^2$ et $R^3$ sont choisis dans le groupe se composant de radicaux alkyles contenant 1 à atomes de carbone et de radicaux haloalkyles contenant 2 à 6 atomes de carbone, pourvu que, lorsque $R^3$ est un groupe haloalkyle, il contienne au moins 3 atomes de carbone, $R^4$ et $R^5$ sont choisis dans le groupe se composant d'hydrogène, de radicaux alkyles et de radicaux haloalkyles où ces radicaux contiennent 1 à 6 atomes de carbone, et le nombre total d'atomes de carbone dans les groupes $R^1$, $R^2$, $R^3$, $R^4$ et $R^5$ ne dépasse pas 14; et l'acide est choisi dans le groupe se composant de:

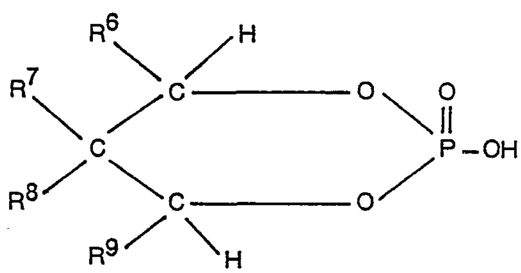 ,

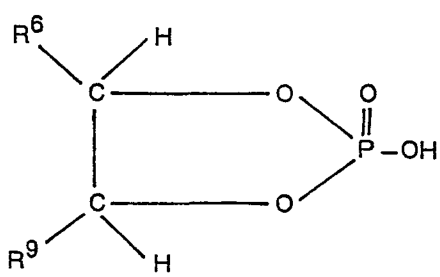 ,

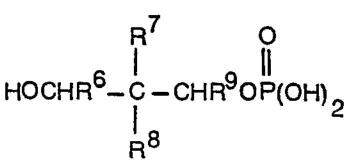 ,

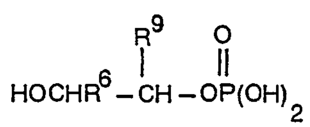 ,

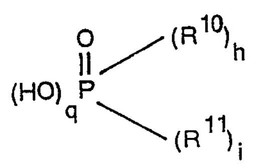 ,

et de leurs mélanges, où $R^6$, $R^7$, $R^8$ et $R^9$ sont indépendamment choisis dans le groupe se composant d'hydrogène, de groupe alkyle ayant 1 à 3 atomes de carbone, haloalkyle ayant 1 à 3 atomes de carbone et hydroxyalkyle ayant 1 à 3 atomes de carbone, pourvu que le nombre total d'atomes de carbone dans les groupes $R^6$, $R^7$, $R^8$ et $R^9$ ne dépasse pas 5; où q est un nombre entier valant 1 à 2; h et i sont des nombres entiers indépendamment choisis dans le groupe se composant de 0, 1 et 2 et q plus h plus i est égal à 3; et où $R^{10}$ et $R^{11}$ sont indépendamment choisis dans le groupe se composant de groupe alkyle ayant 1 à 6 atomes de carbone, haloalkyle ayant 1 à 6 atomes de carbone et 1 à 3 atomes

d'halogène, hydroxy-polyalkylèneoxy contenant 2 à 6 atomes de carbone et 2 à 6 atomes d'oxygène, alcoxy ayant 1 à 6 atomes de carbone, hydroxyalcoxy ayant 2 à 6 atomes de carbone, haloalcoxy ayant 2 à 6 atomes de carbone et 1 à 3 atomes d'halogène,

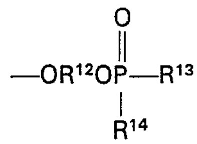

où $R^{12}$ est choisi dans le groupe se composant de groupe alkylène ayant 2 à 6 atomes de carbone, halo-alkylène ayant 3 à 6 atomes de carbone et 1 à 3 atomes d'halogène, polyalkylèneoxy contenant 2 à atomes de carbone et 1 à 5 atomes d'oxygène, et hydroxyalkylène ayant 3 à 6 atomes de carbone et 1 à 4 groupes hydroxyles, et $R^{13}$ et $R^{14}$ sont indépendamment choisis dans le groupe se composant d'hydrogène, de groupe hydroxyle, alkyle ayant 1 à 6 atomes de carbone, haloalkyle ayant 1 à 6 atomes de carbone et 1 à 3 atomes d'halogène, hydroxypolyalkylèneoxy contenant 2 à 6 atomes de carbone, et 2 à 6 atomes d'oxygène, alcoxy ayant 1 à 6 atomes de carbone, hydroxyalcoxy ayant 2 à 6 atomes de carbone, et haloalcoxy ayant 2 à 6 atomes de carbone et 1 à 3 atomes d'halogène, pourvu que le nombre total d'atomes de carbone dans les groupes $R^{10}$ et $R^{11}$ ne dépasse pas 8.

2. Composition intumescente selon la revendication 1, dans laquelle la composition contient 10 à 50% (enpoids) du composé organophosphoré contenant un ou plusieurs groupes hydroxy, 10 à 35% (en poids) d'acide organophosphoré, 30 à 60% (en poids) du composé azoté cyclique et 5 à 50% (en poids, d'eau.

3. Composition intumescente selon la revendication 2, dans laquelle la composition contient 14 à 40% (en poids) du composé organophosphoré contenant un ou plusieurs groupes hydroxy, 14 à 28% (en poids) de l'acide organophosphoré et 8 à 40% (en poids) d'eau.

4. Composition intumescente selon l'une quelconque des revendications 1, 2 ou 3, dans laquelle le nombre total d'atomes de carbone dans les groupes $R^1$, $R^2$, $R^3$, $R^4$ et $R^5$ ne dépasse pas 10.

5. Composition intumescente selon l'une quelconque des revendications précédentes, dans laquelle le composé phosphoré est

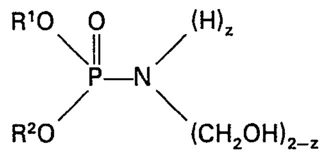

6. Composition intumescente selon l'une quelconque des revendications 1 à 4, dans laquelle de composè phosphoré est

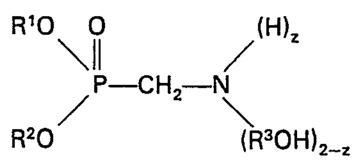

7. Composition intumescente selon l'une quelconque des revendications précédentes, dans laquelle z est 0.

8. Composition intumescente selon l'une quelconque des revendications précédentes, dans laquelle $R^1$ et $R^2$ sont indépendamment choisis dans le groupe se composant de radicaux alkyles contenant 1 à 4 atomes de carbone et de radicaux haloalkyles contenant 2 à 4 atomes de carbone.

9. Composition intumescente selon la revendication 8, dans laquelle $R^1$ et $R^2$ sont indépendamment choisis dans le groupe se composant de groupes éthyle et haloéthyle.

10. Composition intumescente selon la revendication 9, dans laquelle $R^1$ et $R^2$ sont le groupe chloroéthyle.

11. Composition intumescente selon la revendication 9 dans laquelle $R^1$ et $R^2$ sont le groupe éthyle.

12. Composition intumescente selon l'une quelconque des revendications précédentes, dans laquelle l'acide organophosphoré est choisi dans le groupe se composant d'acide 2,2-bis(bromo-méthyl)-3-hydroxy-1-propylphosphorique, de phosphate acide de dibutyle, d'acide mono(tribromo-nèpentyl)phosphorique, de 2-hydroxy-5,5-diméthyle-1,3,2-dioxophosphorinane, d'acide bis(dichloro-isopropyl)phosphorique, d'acide bis(2-chloroéthyl)phosphorique, d'acide chloronéopentylphosphorique et de leurs mélanges.

13. Composition intumescente selon l'une quelconque des revendications précédentes, dans laquelle a plus b plus c vaut 3.

14. Composition intumescente selon la revendication 13, dans laquelle a plus b plus c vaut 6.

15. Composition intumescente selon l'une quelconque des revendications précédentes, dans laquelle X,X' et X'' représentent $CH_3$.

## Patentansprüche

1. Dämmschichtbildendes Mittel enthaltend eine cyclische Stickstoffverbindung, eine hydroxylgruppenhaltige Organophosphorverbindung, eine Organophosphorsäure und Wasser, dadurch gekennzeichnet, daß,

(a) die cyclische Stickstoffverbindung eine Verbindung der Formel

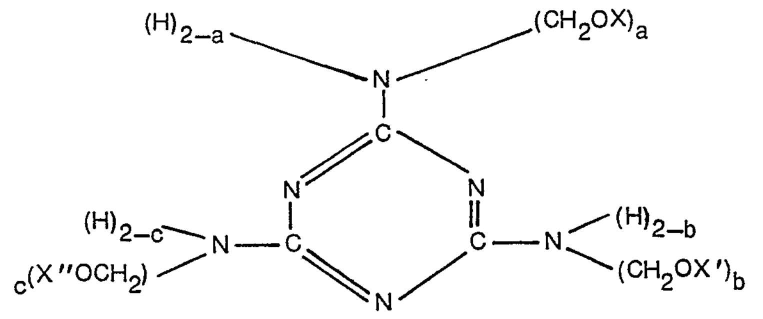

ist, in der a, b und c ganze Zahlen darstellen, die aus der 1 und 2 umfassenden Gruppe ausgewählt sind, a plus b plus c den Wert 3 bis 6 besitzt und X, X' und X'' unabhängig voneinander aus der Wasserstoffatome und $CH_3$-Gruppen umfassenden Gruppe ausgewählt sind;

(b) die hydroxylgruppenhaltige Organophosphorverbindung aus der Gruppe ausgewählt ist, die Verbindungen der Formeln

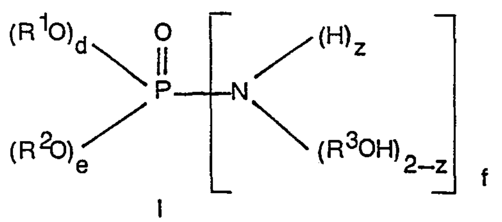

I

und

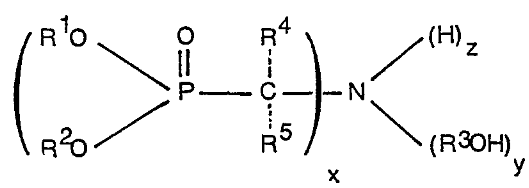

II

und Mischungen davon umfaßt, worin d eine ganze Zahl mit einem Wert von 0 bis 2, e eine ganze Zahl mit einem Wert von 0 bis 2 und f eine ganze Zahl mit einem Wert von 1 bis 3 bedeuten und d plus e plus f den Wert 3 besitzt; z eine ganze Zahl mit einem Wert von 0 bis 1 darstellt; x eine ganze Zahl mit einem Wert von 1 bis 2 und y eine ganze Zahl mit einem Wert von 1 bis 2 bedeuten und x plus y plus z den Wert 3 besitzen; $R^1$, $R^2$ und $R^3$ aus der Gruppe ausgewählt sind, die Alkylgruppen mit 1 bis 6 Kohlenstoffatomen und Halogenalkylgruppen mit 2 bis 6 Kohlenstoffatomen unfaßt, mit der Maßgabe, daß, wenn $R^3$ eine Halogenalkylgruppe darstellt, sie mindestens 3 Kohlenstoffatome aufweist; $R^4$ und $R^5$ aus der Gruppe ausgewählt sind, die Wasserstoffatome, Alkylgruppen und Halogenalkylgruppen umfaßt, wobei diese Gruppen 1 bis 6 Kohlenstoffatome aufweisen; und die Gesamtzahl der Kohlenstoffatome in den Gruppen $R^1$, $R^2$, $R^3$, $R^4$ und $R^5$ 14 nicht übersteigt; und

(c) die Säure aus der Gruppe von Verbindungen der Formeln

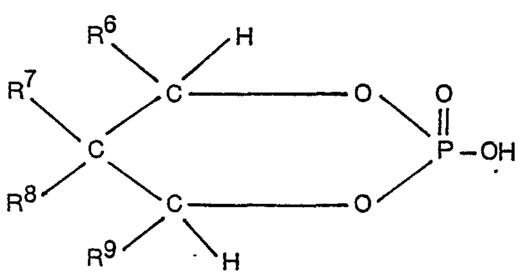

,

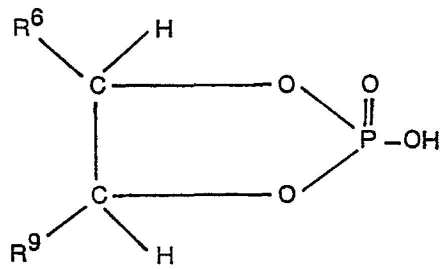

,

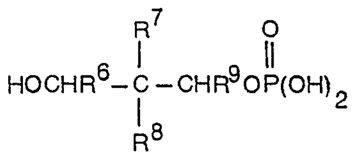, 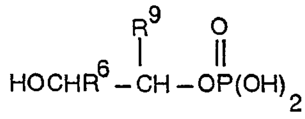, 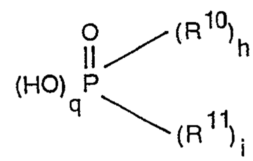,

und Mischungen davon ausgewählt ist, worin $R^6$, $R^7$, $R^8$ und $R^9$ unabhängig voneinander aus der Gruppe ausgewählt sind, die Wasserstoffatome, Alkylgruppen mit 1 bis 3 Kohlenstoffatomen, Halogenalkylgruppen mit 1 bis 3 Kohlenstoffatomen und Hydroxyalkylgruppen mit 1 bis 3 Kohlenstoffatomen umfaßt, mit der Maßgabe, daß die Gesamtanzahl der Kohlenstoffatome in den Gruppen $R^6$, $R^7$, $R^8$ und $R^9$ 5 nicht übersteigt; q eine ganze Zahl mit einem Wert von 1 bis 2 und h und i unabhängig voneinander ganze Zahlen aus der 0, 1 und 2 umfassenden Gruppe darstellen und q plus h plus i den Wert 3 besitzt; und worin $R^{10}$ und $R^{11}$ unabhängig voneinander aus der Gruppe ausgewählt sind, die Alkylgruppen mit 1 bis 6 Kohlenstoffatomen, Halogenalkylgruppen mit 1 bis 6 Kohlenstoffatomen und 1 bis 3 Halogenatomen, Hydroxy-polyalkylenoxygruppen, die 2 bis 6 Kohlenstoffatome und 2 bis 6 Sauerstoffatome umfassen, Alkoxygruppen mit 1 bis 6 Kohlenstoffatomen, Hydroxyalkoxygruppen mit 2 bis 6 Kohlenstoffatomen, Halogenalkoxygruppen mit 2 bis 6 Kohlenstoffatomen und 1 bis 3 Halogenatomen, Gruppen der Formel

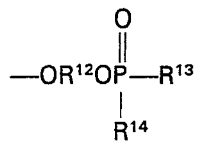

worin $R^{12}$ aus der Gruppe ausgewählt ist, die Alkylengruppen mit 2 bis 6 Kohlenstoffatomen, Halogenalkylengruppen mit 3 bis 6 Kohlenstoffatomen und 1 bis 3 Halogenatomen, Polyalkylenoxygruppen mit 2 bis 6 Kohlenstoffatomen und 1 bis 5 Sauerstoffatomen und Hydroxyalkylengruppen mit 3 bis 6 Kohlenstoffatomen und 1 bis 4 Hydroxylgruppen umfaßt und $R^{13}$ und $R^{14}$ unabhängig voneinander aus der Gruppe ausgewählt sind, die Wasserstoffatome, Hydroxylgruppen, Alkylgruppen mit 1 bis 6 Kohlenstoffatomen, Halogenalkylgruppen mit 1 bis 6 Kohlenstoffatomen und 1 bis 3 Halogenatomen, Hydroxy-polyalkylenoxygruppen mit 2 bis 6 Kohlenstoffatomen und 2 bis 6 Sauerstoffatomen, Alkoxygruppen mit 1 bis 6 Kohlenstoffatomen, Hydroxyalkoxygruppen mit 2 bis 6 Kohlenstoffatomen und Halogenalkoxygruppen mit 2 bis 6 Kohlenstoffatomen und 1 bis 3 Halogenatomen umfaßt, bedeuten, mit der Maßgabe, daß die Gesamtzahl der Kohlenstoffatome in den Gruppen $R^{10}$ und $R^{11}$ 8 nicht übersteigt.

2. Dämmschichtbildendes Mittel nach Anspruch 1, dadurch gekennzeichnet, daß das Mittel 10 bis 50 Gew.-% der hydroxylgruppenhaltigen Organophosphorverbindung, 10 bis 35 Gew.-% der Organophosphorsäure, 30 bis 60 Gew.-% der cyclischen Stickstoffverbindung und 5 bis 50 Gew.-% Wasser enthält.

3. Dämmschichtbildendes Mittel nach Anspruch 2, dadurch gekennzeichnet, daß das Mittel 14 bis 40 Gew.-% der hydroxylgruppenhaltigen Organophosphorverbindung, 14 bis 28 Gew.-% der Organophosphorsäure und 8 bis 40 Gew.-% Wasser enthält.

4. Dämmschichtbildendes Mittel nach den Ansprüchen 1, 2 oder 3, dadurch gekennzeichnet, daß die Gesamtzahl der Kohlenstoffatome in den Gruppen $R^1$, $R^2$, $R^3$, $R^4$ und $R^5$ 10 nicht übersteigt.

5. Dämmschichtbildendes Mittel nach einem der vorhergehenden Ansprüche, dadurch gekennzeichnet, daß die Phosphorverbindung der Formel

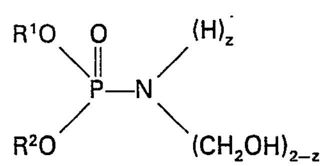

entspricht.

6. Dämmschichtbildendes Mittel nach einem der Ansprüche 1 bis 4, dadurch gekennzeichnet, daß die Phosphorverbindung der Formel

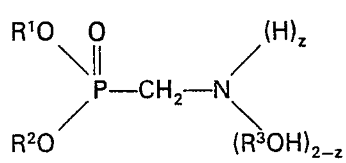

entspricht.

7. Dämmschichtbildendes Mittel nach einem der vorhergehenden Ansprüche, dadurch gekennzeichnet, daß z den Wert 0 besitzt.

8. Dämmschichtbildendes Mittel nach einem der vorhergehenden Ansprüche, dadurch gekennzeichnet, daß die Gruppen $R^1$ und $R^2$ unabhängig voneinander aus der Gruppe ausgewählt sind, die Alkylgruppen mit 1 bis 4 Kohlenstoffatomen und Halogenalkylgruppen mit 2 bis 4 Kohlenstoffatomen unfaßt.

9. Dämmschichtbildendes Mittel nach Anspruch 8, dadurch gekennzeichnet, daß die Gruppen $R^1$ und $R^2$ unabhängig voneinander aus der Ethylgruppen und Halogenethylgruppen amfassenden Gruppe ausgewählt sind.

10. Dämmschichtbildendes Mittel nach Anspruch 9, dadurch gekennzeichnet, daß die Gruppen $R^1$ und $R^2$ Chlorethylgruppen bedeuten.

11. Dämmschichtbildendes Mittel nach Anspruch 9, dadurch gekennzeichnet, daß die Gruppen $R^1$ und $R^2$ Ethylgruppen darstellen.

12. Dämmschichtbildendes Mittel nach einem der vorhergehenden Ansprüche, dadurch gekennzeichnet, daß die Organophosphorsäure aus der Gruppe ausgewählt ist, die 2,2-Bis(brommethyl)-3-hydroxy-1-propylphosphorsäure, Dibutylhydrogenphosphat, Mono-(tribromneopentyl)-phosphorsäure, 2-Hydroxy-5,5-dimethyl-1,3,2-dioxophosphorinan, Bis(dichloroisopropyl)-phosphorsäure, Bis(2-chlorethyl)-phosphorsäure, Chlorneopentylphosphorsäure oder eine Mischung davon ist.

13. Dämmschichtbildendes Mittel nach einem der vorhergehenden Ansprüche, dadurch gekennzeichnet, daß a plus b plus c den Wert 3 besitzt.

14. Dämmschichtbildendes Mittel nach Anspruch 13, dadurch gekennzeichnet, daß a plus b plus c den Wert 6 besitzt.

15. Dämmschichtbildendes Mittel nach einem der vorhergehenden Ansprüche, dadurch gekennzeichnet, daß X, X′ und X″ $CH_3$-Gruppen bedeuten.